# EUROPEAN PATENT APPLICATION

(11) **EP 4 566 975 A1**
(43) Date of publication of application: **11.06.2025**
(21) Application number: 23215006.0
(22) Date of filing: 07.12.2023
(51) Int. Cl.: B65H 51/04, B65H 51/10, A61B 34/30, A61B 1/00, A61M 25/01, A61M 25/09, B65G 39/00

(54) **DRIVE ROLLER FOR ADVANCING AND/OR RETRACTING A CYLINDRICAL OBJECT**

(71) Applicant: Koninklijke Philips N.V., 5656 AG Eindhoven (NL)
(72) Inventor: PATRICIU, Alexandru, Eindhoven (NL); BALICKI, Marcin Arkadiusz, Eindhoven (NL)
(74) Representative: Philips Intellectual Property & Standards

(57) **Abstract**

The invention relates to a drive roller 2 for advancing and/or retracting a cylindrical object 104. The drive roller 2 comprises a core member 6 and a deformable member 8 radially surrounding the core member 6. The deformable member 8 comprises an outer peripheral surface 10. The outer peripheral surface 10 comprises an open notch 12, the open notch 12 being configured to receive the cylindrical object 104 therein. Thereby, the open notch 12 is deformed when the cylindrical object 104 is pushed against a bottom surface of the open notch 12 such that the cylindrical object 104 is better captured by the drive roller 2. As a consequence, very good traction forces are generated by such drive roller 2 while the overall dimensions of a corresponding drive roller assembly 200 or even robotic apparatus 300 are small.

## Description

### FIELD OF THE INVENTION

The invention relates to a drive roller for advancing and/or retracting a cylindrical object, a drive roller assembly and a robotic apparatus.

### BACKGROUND OF THE INVENTION

Drive rollers are used in various applications to advance, retract, and potentially rotate cylindrical objects, such as welding wires, needles, endovascular catheters, endovascular guidewires and the like. In principle, the cylindrical object is engaged between two drive rollers, and, by rotating the drive rollers synchronously in opposite directions, the object is advanced. The maximum translational and torque force is a function of the friction coefficient between the two contact materials of the drive roller, the force applied on the wire and the contact surface.

The local pressure on an endovascular device must be minimized. In order to achieve this, it is proposed in the prior art to utilize multiple rollers that are spatially distributed along a longitudinal axis along the cylindrical object to be advance. However, said design requires a significant contact length between the multiple rollers and the cylindrical object which limits the available working length of the cylindrical object, for example a catheter. As a consequence, the cylindrical object may not reach to the desired location inside the patient, causing delays due to an exchange for a new device and potential complications.

### SUMMARY OF THE INVENTION

It is an object of the present invention to provide a drive roller, a compact drive roller assembly and a robotic apparatus that provide better traction by achieving a better distribution of forces on the advanced or retracted cylindrical object in order to minimize the likelihood of damaging said cylindrical object.

In a first aspect, a driver roller for advancing and/or retracting a cylindrical object is presented. The drive roller comprises a core member and a deformable member radially surrounding the core member, wherein the deformable member comprises an outer peripheral surface and wherein the outer peripheral surface comprises an open notch, the open notch being configured to receive the cylindrical object therein. The deformable member comprises design and/or material properties causing a deformation of a lateral surface of the notch when the tubular object is pushed against a bottom surface of the notch.

It has been found that a lateral surface of the open notch is deformed when the cylindrical object is pushed against a bottom surface of the open notch such that the cylindrical object is better captured by the drive roller as a kind of squeezing. The friction force between the cylindrical object and the roller is increased due to the increased contact surface and the resulting normal contact force. As a consequence, very good traction forces are generated by such drive roller while the overall dimensions of a corresponding drive roller assembly or even robotic apparatus are small.

In a second aspect, a drive roller for advancing and/or retracting a cylindrical object is presented. The drive roller according to the second aspect comprises a core member, a deformable member radially surrounding the core member, and an elastic outer member radially surrounding the deformable member, wherein the deformable member comprises a rigidity that is lower than a rigidity of the elastic outer member.

It has been found that by utilizing a deformable member being more deformable than the elastic outer member, and by utilizing a more rigid core member, the deformable member allows a deformation of the whole drive roller under radial pressure, while the more rigid elastic outer member allows a firm holding of the cylindrical object without any significant tolerance with regard to an outer peripheral surface of the elastic outer member. Thus, no significant local movements of the cylindrical object are possible along the drive rollers thereby avoiding a local overstress on the outer peripheral surface or an unequal property of a deformable material on the surface. In other words, the whole drive roller is deformed due to the deformable member since the pressure is thus imparted on the whole outer peripheral surface. Thereby, the contact surface between the drive roller and the cylindrical object is maximized while the local force exerted on the cylindrical object is minimized. Hence, very good traction forces even on small cylindrical objects are achieved.

In one embodiment, the rigidity of the deformable member is adjusted by means of varying the material properties of the deformable member. The rigidity of the deformable member may additionally or alternatively be adjusted by means of varying the design properties of the deformable member. For example, the deformable member may comprise spokes or other three-dimensional structures that provide the required rigidity properties.

In a preferred embodiment, the elastic outer member comprises an outer peripheral surface, wherein the outer peripheral surface comprises an open notch, the open notch configured to receive the cylindrical object therein. The open notch ensures that the cylindrical object is properly guided with respect to the drive roller. When the cylindrical object is inserted into the open notch, the deformable member is compressed together with the elastic outer member, and the cylindrical object is trapped in the open notch of the drive roller and a corresponding notch of a second drive roller.

The deformable member preferentially comprises a rigidity that is lower than a rigidity of the core member. Thereby, the core member may transfer the rotation, for example, via a drive shaft, wherein an outside surface of the core member is designed or arranged to receive the deformable member. Thus, due to its higher rigidity, the core member is capable of transferring a torque or rotation, wherein the deformable member has a higher rigidity in order to maximize the contact surface between the elastic outer member or the deformable member itself and the cylindrical object.

In a preferred embodiment, the open notch comprises two side surfaces that are tapered towards one another and a bottom surface connecting the two side surfaces, wherein the side surfaces of the open notch are deformed when the cylindrical object is pushed against the bottom surface of the open notch. Thus, the open notch is designed in such a way that it corresponds to the outer diameter of the cylindrical object, wherein by means of the tapered walls, the cylindrical object is slightly squeezed into the open notch.

The bottom surface of the open notch may comprises a curvature. In particular, the curvature corresponds to a curvature of the cylindrical object to be advanced or retracted. This maximizes the contact surface between the drive roller and the cylindrical object while minimizing the local forces exerted onto the cylindrical object. Furthermore, good traction forces are achieved even for cylindrical objects having a small diameter.

Preferentially, the outer peripheral surface is configured as a toothed outer peripheral surface. According to a preferred embodiment, the open notch is arranged at outer radial edges of the toothed outer peripheral surface. In this way, two drive rollers may engage with one another. When a cylindrical object is inserted, the toothed outer peripheral surface is compressed, and the cylindrical object is trapped in the two open notches of the two gear-like drive rollers that are meshing. Furthermore, due to the local deformation of the toothed outer peripheral surface, the drive rollers will start behaving like a belt and there will be more than one tooth engaged on the longitudinal axis of the cylindrical object at the same time. This increases the contact surface of the area between the cylindrical object and the drive rollers providing better traction and lower pressures in the individual contact areas between the drive rollers and the cylindrical object. In addition, the toothed outer peripheral surfaces engage with the other drive roller increasing power transfer synchronization between the two rotating drive rollers. This prevents the cylindrical object itself from being used as a power coupling between the two drive rollers. The height of the toothed outer peripheral surface may be used to control the maximum compressive force when the teeth of the toothed outer peripheral surface bottom out on the tooth of the opposite drive roller.

The drive roller may comprise a ring, the ring comprising protrusions configured to push the cylindrical object into the open notch of the drive roller. By means of the ring, the cylindrical object can be pushed into the open notch of the pairing drive roller in order to ensure good traction.

The deformable member preferentially comprises an outer profile, wherein the outer profile comprises protrusions. Said protrusions help pushing the cylindrical object into the paired drive roller.

According to an embodiment, the deformable member is made of or comprises at least one of the following materials: Silicone rubber, Nitrile rubber, or other types of rubbers. Silicone Rubber has been found to be beneficial in order to provide the required rigidity of the deformable member.

According to one embodiment, the core member is made of or comprises at least one of the following materials: Polycarbonate, ABS (Acrylonitrile butadiene styrene), Acrylic, or Metal. Preferentially, the elastic outer member is made of or comprises at least one of the following materials: HDPE (High Density Polyethylen), POM (Polyoxymethylene), Polyethylene. The mentioned materials for the core member and the elastic outer member have been found to be beneficial for a power transmission from the drive rollers to the cylindrical object, while the local force exerted onto the cylindrical object is minimized.

According to a preferred embodiment, at least one of the deformable member, the core member or the elastic outer member comprise features to ensure power transmission between the respective adjacent member. In this way, the transfer of power and torque between the core member, the elastic outer member and the deformable member may be improved.

According to one embodiment, the drive roller or at least one of the core member and/or the deformable member and/or the elastic outer member may be 3D-printed. By means of the printed geometry of the members, the rigidity of the members may also be adjusted.

According to a preferred embodiment, the drive roller comprises a hinge arranged at the bottom of the open notch, wherein the hinge is formed in the elastic outer member and is arranged adjacent to the deformable member. In this way, when the cylindrical object is pushed into the open notch, the hinge will allow the open notch to close squeezing the cylindrical object inside. In other words, the deformable member allows for the deformation of the elastic outer member comprising the toothed outer peripheral surface. Since a side layer of the toothed elastic outer member has a smaller thickness, it will be stiffer than the inner part thereof. When the cylindrical object is pressed into the open notch, the toothed outer peripheral surface will deform and thus close the notch and trap the cylindrical object inside. Thus, the open notch closes as the cylindrical object is pressed in effectively trapping and squeezing the cylindrical object therein. This results in good force distribution around the cylindrical object and very high normal forces providing better traction than flat or simple profiled surfaces. Thus, the hinge allows for a small deformation of the gear teeth when a cylindrical object is loaded, resulting in high normal forces on the cylindrical object.

The deformable member is preferably molded onto the core member. Alternatively, the deformable member comprises at least one O-ring, in particular two O-rings, that are coaxially arranged with respect to an axis of rotation of the drive roller. Therewith, an alternative way of establishing the deformable member is presented.

In another aspect of the present invention, a drive roller assembly is presented. The drive roller assembly comprises a pair of drive rollers, the drive rollers comprising outer peripheral surfaces facing one another and being configured to accommodate a cylindrical object there between, and a translation mechanism configured to drive both drive rollers, so as to advance or retract a cylindrical object arranged between the drive rollers, wherein one or both of the drive rollers are configured according to any one of claims 1 to 11.

In another aspect of the present invention, a drive roller assembly is presented. The drive roller assembly comprises a pair of drive rollers, the drive rollers comprising outer peripheral surfaces facing one another and being configured to accommodate a cylindrical object there between, a translation mechanism configured to drive a first one of the drive rollers, wherein a second one of the drive rollers is configured to freely rotate about its axis of rotation, wherein one or both of the drive rollers are configured according to any one of claims 1 to 11.

Preferentially, the second drive roller is positioned above the first drive roller. In this way, the drive roller assembly may squeeze a cylindrical object between the drive rollers, wherein one of the drive rollers is arranged to be rotated by the translation mechanism, for example, by means of an external actuator, such as a motor, and the second one of the drive rollers may rotate freely around its axis of rotation. By positioning the second drive roller above the first drive roller, the first drive roller is pressed down by gravity with a certain force such that the cylindrical object is pressed between the elastic outer members of the drive rollers. This is in particular beneficial for providing high traction forces for cylindrical objects having a small diameter.

In another aspect of the present invention, a robotic apparatus, in particular, an endovascular robotic apparatus, is presented. The apparatus comprises a roller assembly according to any one of claims 12 to 14. Preferentially, the robotic apparatus further comprises a rotating mechanism configured to rotate the cylindrical object and/or the roller assembly.

It shall be understood that the drive roller claims 1 to 12, the drive roller assembly of claims 13 and 14 and the robotic apparatus of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of a drive roller for advancing and/or retracting a cylindrical object,
Fig. 2 shows schematically and exemplarily an alternative embodiment of a drive roller for advancing and/or retracting a cylindrical object,
Fig. 3 illustrates the embodiment of Fig. 2 in a sectional view,
Fig. 4 shows schematically and exemplarily an alternative embodiment of a drive roller for advancing and/or retracting a cylindrical object,
Fig. 5a illustrates the embodiment of Fig. 4 in a sectional view,
Fig. 5b illustrates the working principle of the hinge and the open notch of the embodiment of Figs. 4 and 5a,
Fig. 5c illustrates the deformation of the toothed outer peripheral surface of the embodiment of Figs. 4, 5a and 5b when a cylindrical object is pressed into the notch,
Fig. 6 shows schematically and exemplarily an alternative embodiment of a drive roller for advancing and/or retracting a cylindrical object,
Fig. 7 illustrates the embodiment of Fig. 6 in a sectional view,
Fig. 8 shows schematically and exemplarily an alternative embodiment of a drive roller for advancing and/or retracting a cylindrical object in a perspective view,
Fig. 9 shows schematically and exemplarily another embodiment of a drive roller for advancing and/or retracting a cylindrical object in a perspective view,
Fig. 10 shows schematically and exemplarily an embodiment of a robotic apparatus,
Fig. 11 shows schematically and exemplarily an alternative embodiment of a robotic apparatus.

### DETAILED DESCRIPTION OF EMBODIMENTS

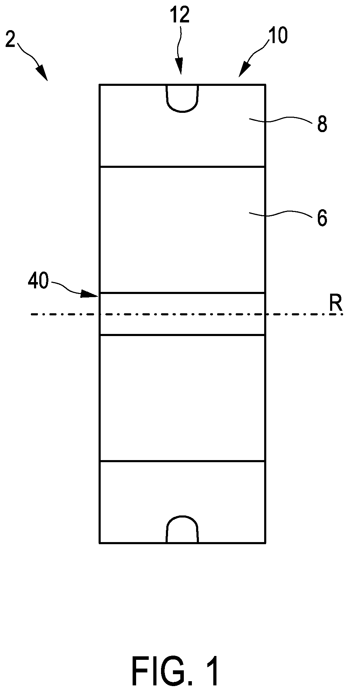

Fig. 1 shows a drive roller 2 for advancing and/or retracting a cylindrical object (not shown in Fig. 1). The drive roller 2 comprises a core member 6. The drive roller 2 furthermore comprises a deformable member 8 radially surrounding the core member 6. The deformable member 8 comprises an outer peripheral surface 10. The outer peripheral surface 10 comprises an open notch 12. The open notch 12 is configured to receive the cylindrical object therein. The drive roller 2 comprises a central bore 40 configured to receive a driving means therein. The drive roller 2 is configured to rotate around an axis of rotation R. The open notch 12 extends along the outer peripheral surface 10, wherein the open notch 12 is arranged in the middle of the drive roller 2 seen along the axis of rotation R. It has been found that the open notch 12 is deformed when the cylindrical object is pushed against a bottom surface of the open notch 12 such that the cylindrical object is better captured by the drive roller 2 as a kind of squeezing. The friction force between the cylindrical object and the drive roller 2 is increased due to the increased contact surface and the resulting normal contact force. As a consequence, very good traction forces are generated by such drive roller 2.

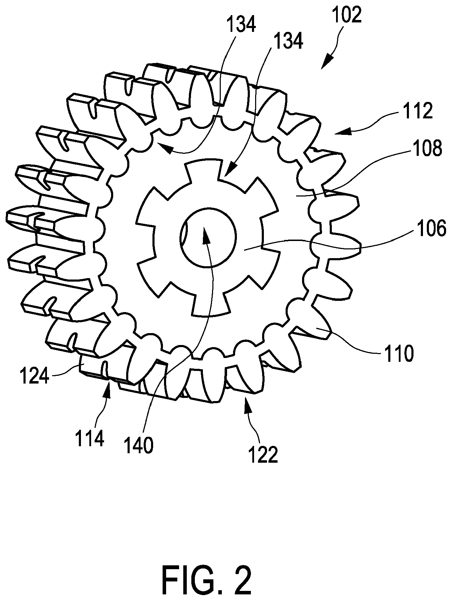

Fig. 2 shows another embodiment of a drive roller 102. The drive roller 102 comprises a core member 106. The core member 106 is radially surrounded by a deformable member 108. An elastic outer member 110 radially surrounds the deformable member 108. The deformable member 108 comprises a rigidity that is lower than a rigidity of the elastic outer member 110.

By utilizing a deformable member 108 being more deformable than the elastic outer member 110, and by utilizing a more rigid core member 106, the deformable member 108 allows a deformation of the whole drive roller 102 under radial pressure, while the more rigid elastic outer member 110 allows a firm holding of the cylindrical object without any significant tolerance with regard to an outer peripheral surface 122 of the elastic outer member 110.

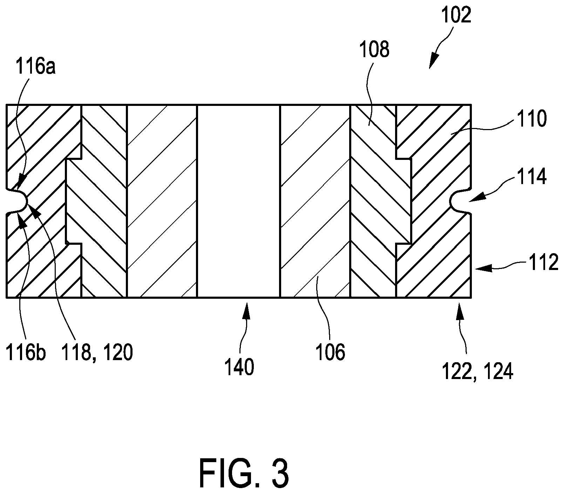

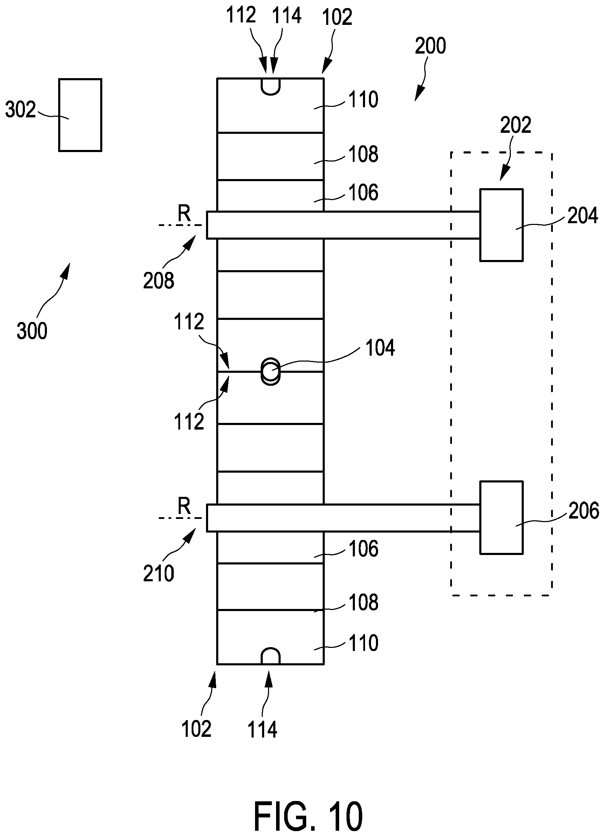

The elastic outer member 110 comprises the outer peripheral surface 112. The outer peripheral surface 112 is configured as a toothed outer peripheral surface 122, wherein the toothed outer peripheral surface 122 comprises an open notch 114. The open notch 114 is configured to receive a cylindrical object 104 (see Fig. 10) therein. The deformable member 108 comprises a rigidity that is lower than a rigidity of the core member. The open notch 114 comprises, as shown in Fig. 3, two side surfaces 116a, 116b. The side surfaces 116a, 116b are tapered towards one another. The open notch 114 furthermore comprises a bottom surface 118 connecting the two side surfaces 116a, 116b. The side surfaces 116a, 116b of the open notch 114 are deformed when the cylindrical object 104 is pushed against the bottom surface 118 of the open notch 114. The bottom surface 118 of the open notch 114 comprises a curvature 120 which corresponds to an outside diameter of a cylindrical object 104 (not shown in Fig. 3). The open notch 114 is arranged at outer radial edges 124 of the toothed outer peripheral surface 122. Furthermore the deformable member 108, the core member 106 and the elastic outer member 110 comprise features 134 that ensure power transmission between the respective adjacent members 106, 108 and 110.

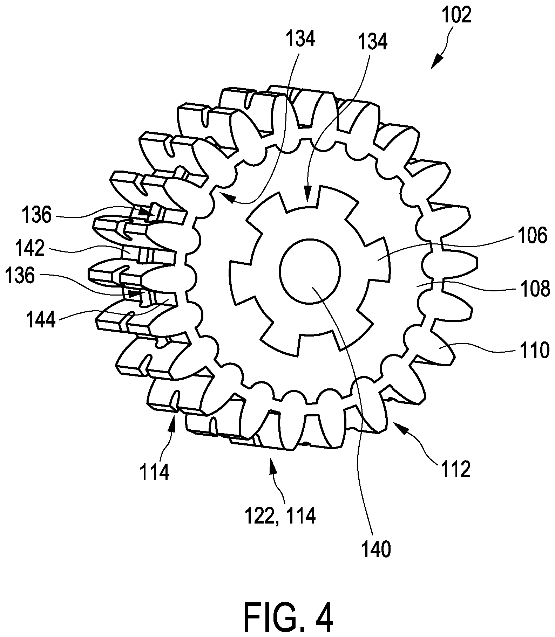

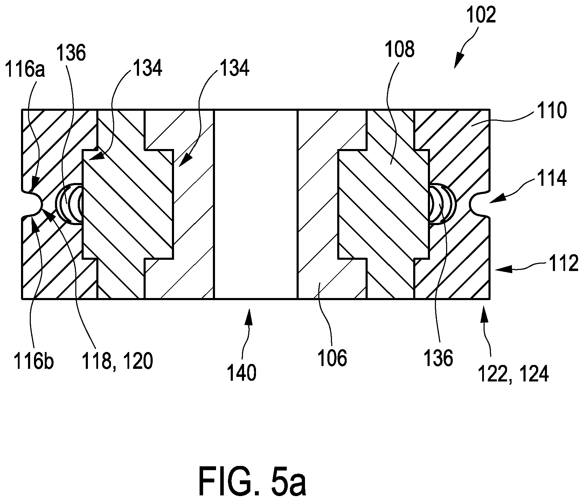

A further embodiment of a drive roller 102 is shown in Figs. 4 and 5a-c. The drive roller 102 is similar to the one shown in Figs. 2 and 3 and comprises a core member 106, a deformable member 108 and an elastic outer member 110. Furthermore, the elastic outer member 110 comprises an outer peripheral surface 112, wherein the outer peripheral surface 112 comprises an open notch 114, the open notch 114 being configured to receive the cylindrical object 104 therein. As shown in Fig. 5a, the open notch 114 comprises two side surfaces 116a, 116b that are tapered towards one another and a bottom surface 118 connecting the two side surfaces 116a, 116b. The side surfaces 116a, 116b of the open notch 114 are deformed when the cylindrical object 104 is pushed against the bottom surface 118 of the open notch 114. The bottom surface 118 of the open notch 114 comprises a curvature 120. The outer peripheral surface 112 is configured as a toothed outer peripheral surface 122. The open notch 114 is arranged at outer radial edges 124 of the toothed outer peripheral surface 122. The deformable member 108, the core member 106 and the elastic outer member 110 comprise features 134 that ensure power transmission between the respective adjacent members 106, 108 and 110.

The embodiment of Figs. 4 and 5a-c additionally comprises - compared to the embodiment of the previous figures - a hinge 136. The hinge 136 is arranged at the bottom of the open notch 114. The hinge 136 is formed in the elastic outer member 110 and is arranged adjacent to the deformable member 108. In a direction along the axis of rotation R, the hinge is arranged in the middle of the drive roller 102. The hinge 136 separates the elastic outer member into two rings 142, 144, having the hinge 136 there between. When a cylindrical object 104 (not shown) is pushed into the open notch 114, the hinge 136 will allow the open notch 114 to close, thereby squeezing the cylindrical object 104 inside.

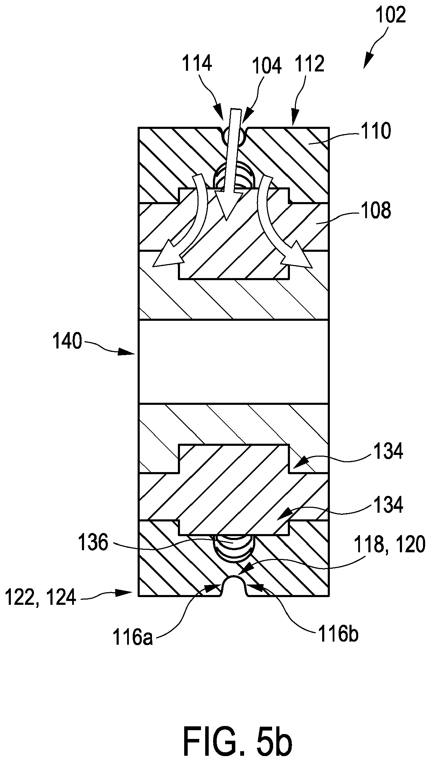

Fig. 5b illustrates the working principle of the hinge 136. When a cylindrical object 104 is pushed into the open notch 114, a compression force is applied to the elastic outer member 110. Since the elastic outer member 110 has a smaller thickness, it will be stiffer than the deformable member 108. When the cylindrical object 104 is pressed into the open notch 114, the toothed elastic outer member 110 will deform and will at least partly close the open notch 114 and trap the cylindrical object 104 inside.

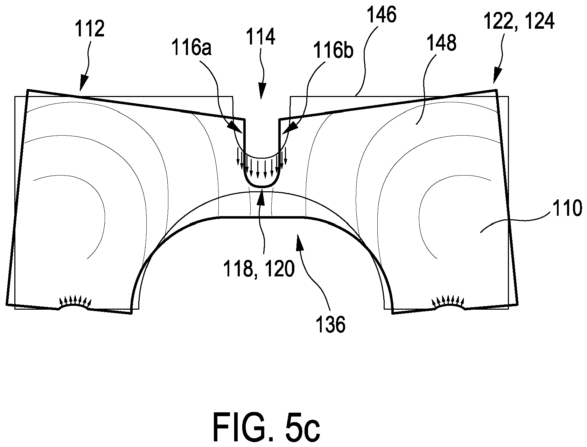

Fig. 5c illustrates the deformation of the toothed outer peripheral surface 122 of the embodiment of Figs. 4, 5a and 5b when a cylindrical object 104 is pressed into the notch. In particular, Fig. 5c shows an undeformed configuration 146 of the toothed outer peripheral surface 122 and a deformed configuration 148. It can be observed that the open notch 114 at least partly closes as the cylindrical object 104 is pressed in the open notch 114 effectively trapping and squeezing the cylindrical object 104 therein. This results in good force distribution around the cylindrical object 104 surface and very high normal forces providing much better traction than flat or simple profiled surfaces. The hinge 136 at the bottom of the elastic outer member 110 allows for a small deformation of the toothed outer peripheral surface 122 when a cylindrical object 104 is loaded resulting in high normal forces on the cylindrical object 104.

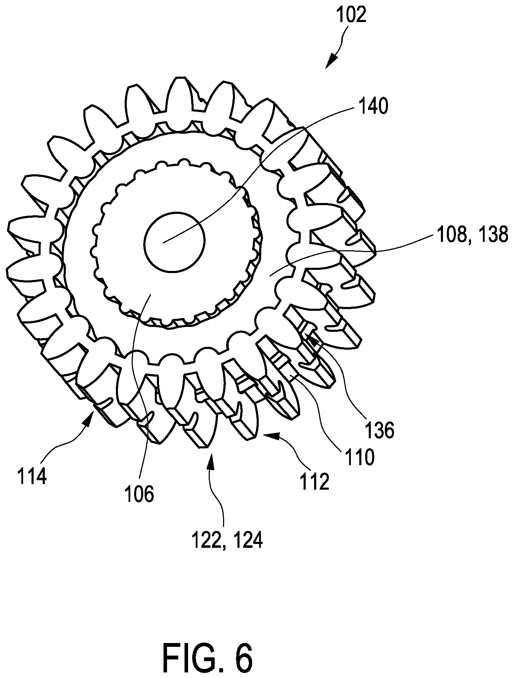

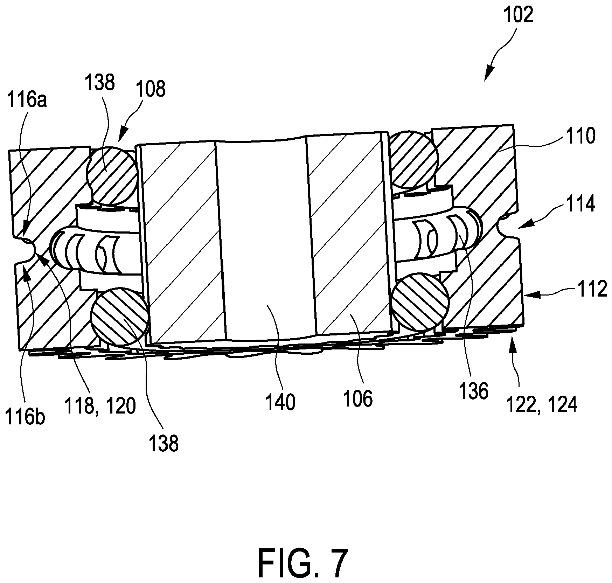

Figs. 6 and 7 show an alternative embodiment of a drive roller 102 comprising a core member 106 and an elastic outer member 110. The elastic outer member 110 is similar to the one of Figs. 4 and 5. The core member 106 comprises a toothed outer geometry, wherein the deformable member comprises two O-rings 138 that are coaxially arranged with respect to the axis of rotation R of the drive roller 102. The core member 106 comprises a toothed outer geometry that ensures power transmission between the core member 106 and the deformable member 108 which comprises the two O-rings 138.

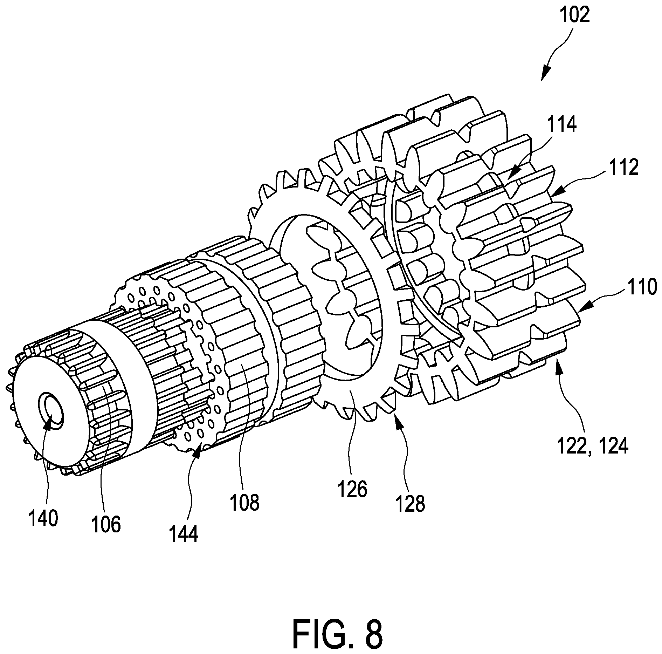

Fig. 8 shows yet another alternative embodiment of a drive roller 102. The driver roller 102 comprises a core member 106, a deformable member 108 and an elastic outer member 110. The elastic outer member 110 comprises an open notch 114 that extends along an outer peripheral surface 112. The elastic outer member 110 comprises a toothed outer peripheral surface 122, wherein the open notch 114 is arranged at outer radial edges 124 of the toothed outer peripheral surface 122. In addition to the previous embodiments, the embodiment of Fig. 8 comprises a ring 126 comprising protrusions 128. The protrusions 128 extend radially from the ring 126. The drive roller 102 of the embodiment of Fig. 8 is in particular helpful for advancing and/or retracting cylindrical objects that are configured as hydrophilic coated guidewires. The stiffness of the deformable member 108 is modulated by adding through holes extending axially there through. The ring 126 will push a cylindrical object 104 (not shown) into another drive roller 102 that forms a pair with the drive roller 102 shown in Fig. 8.

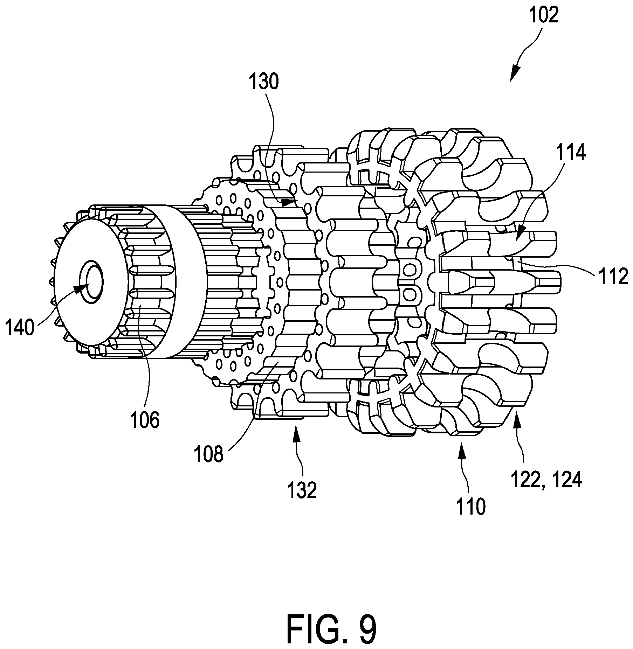

In the embodiment of Fig. 9, a drive roller 102 is shown comprising a core member 106 having a central bore 140 running there through, as well as a deformable member 108 and an elastic outer member 110. The elastic outer member 110 comprises an open notch 114 as well as an outer peripheral surface 112 being configured as a toothed outer peripheral surface 122. The open notch 114 is arranged in outer radial edges 124 of the toothed outer peripheral surface 122. Compared to the previous embodiments, the embodiment of Fig. 9 comprises an outer profile 130 at the deformable member 108. The outer profile 130 comprises protrusions 132. These protrusions 132 help pushing a cylindrical object 104 (not shown) into a second drive roller 102 forming a pair with the drive roller 102 shown in Fig. 9.

Fig. 10 shows a robotic apparatus 300. The robotic apparatus 300 can be an endovascular robotic apparatus. The apparatus 300 comprises a roller assembly 200. The robotic apparatus 300 furthermore may comprise a rotating mechanism 302 configured to rotate a cylindrical object 104 and/or the roller assembly 200. The roller assembly 200 comprises a pair of drive rollers 102. The drive rollers 102 comprise outer peripheral surfaces 112 facing one another and being configured to accommodate the cylindrical object 104 there between. In addition, the drive rollers 102 comprise a core member 106, a deformable member 108 and an elastic outer member 110 which comprises an open notch 114. The drive roller assembly 200 furthermore comprises a translation mechanism 202.

The translation mechanism 202 may be arranged to drive the rotation of at least one of the drive rollers 102 via the core member 106 due to a mechanical connection between the two. Thanks to the rotation of the drive rollers 102 in opposite directions, the cylindrical object 104 provided in the open notch 114 is driven in translation, wherein it depends on the direction of rotation of the drive rollers 102 whether the cylindrical object 104 is retracted or advanced.

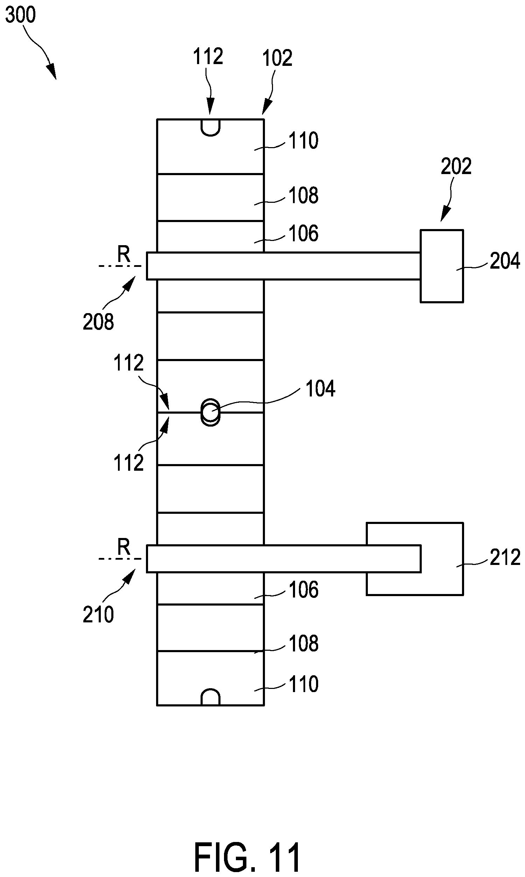

In one particular embodiment, the translation mechanism 202 comprises a shaft connected to the core member 106 of each drive roller 102. It may also comprise one motor to drive in rotation a shaft of a first drive roller 102. In that case, the drive roller 102 is an active drive roller 102, while the second drive roller 102 is a passive drive roller 102 driven by the rotation of the active drive roller 102 via driving elements such as e.g. teeth as shown in Fig. 11. In another embodiment, the translation mechanism 202 may further comprise a second motor connected to the second drive roller 102 via a second shaft or may comprise a motor driving the two drive rollers 102, as shown in Fig. 10.

Fig. 11 shows an alternative embodiment of a robotic apparatus 300 comprising a roller assembly 200. Compared to the embodiment of Fig. 10, the embodiment of Fig. 11 comprises a translation mechanism 202 that is configured to drive a first one 208 of the drive rollers 102, wherein a second one of the drive rollers 102 is configured to freely rotate about its axis of rotation R. Again, the drive rollers 102 may be configured according to any one of the previous embodiments.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like providing a first chromatogram under a first chromatographic condition and providing a second chromatogram under a second chromatographic condition, or the mentioned identification or determination steps performed by one or several units or devices can be performed by any other number of units or devices. These procedures can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.
The invention relates to a drive roller for advancing and/or retracting a cylindrical object. The drive roller comprises a core member and a deformable member radially surrounding the core member. The deformable member comprises an outer peripheral surface. The outer peripheral surface comprises an open notch, the open notch being configured to receive the cylindrical object therein. Thereby, the open notch is deformed when the cylindrical object is pushed against a bottom surface of the open notch such that the cylindrical object is better captured by the drive roller. As a consequence, very good traction forces are generated by such drive roller while the overall dimensions of a corresponding drive roller assembly or even robotic apparatus are small.

## Claims

1. Drive roller (2) for advancing and/or retracting a cylindrical object (104), the drive roller (2) comprising:
- a core member (6),
- a deformable member (8) radially surrounding the core member (6), wherein the deformable member (8) comprises an outer peripheral surface (10) and wherein the outer peripheral surface (10) comprises an open notch (12), the open notch (12) being configured to receive the cylindrical object (104) therein, wherein the deformable member comprises design and/or material properties causing a deformation of a lateral surface of the notch when the tubular object is pushed against a bottom surface of the notch.

2. Drive roller (102) for advancing and/or retracting a cylindrical object (104), the drive roller (102) comprising:
- a core member (106),
- a deformable member (108) radially surrounding the core member (106),
- an elastic outer member (110) radially surrounding the deformable member (108),
wherein the deformable member (108) comprises a rigidity that is lower than a rigidity of the elastic outer member (110).

3. The drive roller (102) as defined by claim 2, wherein the elastic outer member (110) comprises an outer peripheral surface (112) and wherein the outer peripheral surface (112) comprises an open notch (114), the open notch (114) configured to receive the cylindrical object (104) therein.

4. The drive roller (102) as defined by any one of claims 1 to 3, wherein the deformable member (108) comprises a rigidity that is lower than a rigidity of the core member (106).

5. The drive roller (102) as defined by any one of the preceding claims, wherein the open notch (114) comprises two side surfaces (116a, 116b) that are tapered towards one another and a bottom surface (118) connecting the two side surfaces (116a, 116b), and wherein the side surfaces (116a, 116b) of the open notch (114) are deformed when the cylindrical object (104) is pushed against the bottom surface (118) of the open notch (114), in particular wherein the bottom surface (118) of the open notch (114) comprises a curvature (120).

6. The drive roller (102) as defined by any one of claims 3 to 5, wherein the outer peripheral surface (112) is configured as a toothed outer peripheral surface (122), in particular wherein the open notch (114) is arranged at outer radial edges (124) of the toothed outer peripheral surface (122).

7. The drive roller (102) as defined by any one of claims 2 to 6, further comprise a ring (126), the ring (126) comprising protrusions (128) configured to push the cylindrical object (104) into the open notch (114) of the drive roller (102) and/or wherein the deformable member (108) comprises an outer profile (130), and wherein the outer profile (130) comprises protrusions (132).

8. The drive roller (102) as defined by any of the preceding claims, wherein the deformable member (108) is made of or comprises at least one of the following materials:
- Silicone rubber,
- Nitrile rubber,
and/or wherein the core member (106) is made of or comprises at least one of the following materials:
- Polycarbonate,
- ABS (Acrylonitrile butadiene styrene),
- Acrylic,
- Metal,
and/or wherein the elastic outer member (110) is made of or comprises at least one of the following materials:
- HDPE (High Density Polyethylen),
- POM (Polyoxymethylene),
- Polyethylene.

9. The drive roller (102) as defined by any of the preceding claims, wherein at least one of the deformable member (108), the core member (106) or the elastic outer member (110) comprise features (134) to ensure power transmission between the respective adjacent member (106, 108, 110).

10. The drive roller (102) as defined by any one of claims 3 to 9, further composing a hinge (136) arranged at the bottom of the open notch (114), wherein the hinge (136) is formed in the elastic outer member (110) and is arranged adjacent to the deformable member (108).

11. The drive roller (102) as defined by any one of claims 2 to 10,
wherein the deformable member (108) is moulded onto the core member (106), or wherein the deformable member (108) comprises at least one O-ring (138), in particular two O-rings (138) that are coaxially arranged with respect to an axis of rotation (R) of the drive roller (102).

12. Drive roller assembly (200), comprising:
- a pair of drive rollers (102), said drive rollers (102) comprising outer peripheral surfaces (112) facing one another and being configured to accommodate a cylindrical object (104) therebetween,
- a translation mechanism (202) configured to drive both drive rollers (102), so as to advance and/or retract a cylindrical object (104) arranged between the drive rollers (112),
wherein one or both of the drive rollers (112) are configured according to any one of the preceding claims.

13. Drive roller assembly (200), comprising:
- a pair of drive rollers (102), said drive rollers (102) comprising outer peripheral surfaces (112) facing one another and being configured to accommodate a cylindrical object (104) therebetween,
- a translation mechanism (202) configured to drive a first one (208) of the drive rollers (102), wherein a second one (210) of the drive rollers (102) is configured to freely rotate about its axis of rotation (R), wherein one or both of the drive rollers (102) are configured according to any one of claims 1 to 12.

14. Roller assembly (200) as defined by claim 13, wherein the second drive roller (210) is positioned above the first drive roller (208).

15. Robotic apparatus (300), in particular endovascular robotic apparatus, the apparatus (300) comprising a roller assembly (200) according to any one of claims 12 - 14, in particular wherein the robotic apparatus (300) further comprises a rotating mechanism (302) configured to rotate the cylindrical object (104) and/or the roller assembly (200).
